# EUROPEAN PATENT SPECIFICATION

(11) **EP 1 857 557 B1**
(45) Date of publication and mention of the grant of the patent: **16.06.2010**
(21) Application number: 07106801.9
(22) Date of filing: 08.12.2000
(51) Int. Cl.: C12N 15/82, A01H 5/10

(54) **A process for converting storage reserves of dicot seeds into compositions comprising one or more gene products**
Verfahren zur Umformung von Reservestoffen aus zweikeimblättrigen Pflanzensamen in Zusammensetzungen unter Verwendung von einem oder mehreren Genprodukten
Processus de transformation de réserves de stockage de granes de dicotylédone en compositions comprenant un ou plusieures produits géniques

(30) Priority: 10.12.1999 FI 992659
(43) Date of publication of application: 21.11.2007
(62) Divisional of application: 00987501.4
(73) Proprietor: Unicrop Oy, 00790 Helsinki (FI)
(72) Inventor: Kuvshinov, Viktor, 01280 Vantaa (FI); Kanerva, Anne, 00550 Helsinki (FI); Koivu, Kimmo, 00550 Helsinki (FI); Pehu, Eija, 00100 Helsinki (FI)
(74) Representative: Lönnqvist, Gunnel Solveig Kristina

(56) References cited:
- WO-A-94/11519
- WO-A-97/32986
- US-A- 5 994 628

## Description

### The Technical Field of the Invention

The present invention is related to a process based on a source-sink principle for converting the storage reserves of transgenic dicot seeds into a composition comprising one or more gene products of interest. The invention also discloses a germinating seed derived composition comprising at least one gene product in the cotyledon including the seedling or in the medium surrounding the germinating seed or seedling.

### The Background of the Invention

Methods for producing therapeutically active mammalian proteins by isolation and purification from mammalian sources, have nowadays due to an increased contamination risk, largely been replaced by recombinant DNA technology providing new means for producing large amounts of industrially desirable mammalian proteins. Recombinant DNA technology provides a large choice of hosts and expression systems. When mammalian proteins are the desired target proteins, eukaryotic host systems are preferred in order to obtain glycosylated forms of the desired proteins.

Fungi represent the most effective host system for high volume and low cost production of glycosylated proteins. Even if fungi have been successful for producing high amounts of their native proteins, they have not been equally successful in expressing heterologous proteins, such as therapeutic mammalian proteins.

Fungi are nowadays mainly used for large scale production of homologous industrial enzymes, whereas the focus of research has been turned on finding alternative expression systems for producing glycosylated heterologous proteins. Because glycosylation patterns and folding processes in plant host systems resemble those in mammalian systems, plant host expression systems are by far the most cost-effective of the available systems. The main interest has been directed on production of pharmaceuticals and industrially important enzymes. The production of interferon, enkephalins, epidermal growth factor and human serum albumin in tobacco, and/or potato can be mentioned. The expression levels in transgenic plants have been rather low.

In plants, foreign genes are generally expressed under strong tissue specific plant promoters in developing plant organs. Typical examples are the seed storage protein promoter or the tuber specific patatin promoter in potato tubers. Alternatively, cell or organ cultures and algal cultures are used as well as even more sophisticated systems including plant virus based systems, with the desired gene coupled inside the virus genome and expressed concurrently with viral proteins. The most advanced systems rely on inducible expression and secretion of recombinant protein to the medium.

The patents US 5,693,506 and US 5,994,628 disclose a production system for producing foreign proteins in germinating monocot seeds. Recombinant proteins are expressed under a strong amylase promoter in a cell culture or in germinating seed and the protein is secreted into the growth medium or extracted from malt. In a monocot seed the storage reserves mainly consist of the starchy endosperm. The protein stores in monocots are scarce as compared to those in dicot seeds. In addition, even if the amylase expression is high in specific cells, only a small number of cells in the seed express amylase. These cells are restricted to the scutellum of the embryo and the aleurone layer of the endosperm. There is a great demand to provide more cost effective systems, which would take advantage of the storage reserves of proteins and oils in dicot seeds. The patents US 5,543,576 and US 5,714,474 disclose a method in which transgenic seeds are added without any pregermination and in ground form into feed mixtures as additional enzyme sources.

The patent US 5,670,349 discloses the use of wound inducible HMGR/HMG2 promoters for expressing recombinant proteins in fresh tobacco leaves harvested from the field. Wounding of fresh or stored plant material by excision or crushing triggers a rapid increase in expression. However, the protein content in tobacco leaf is small compared to that obtainable from a dicot seed. Furthermore, the storability of fresh leaves is not comparable to that of dry seed in respect of time, space and/or storage costs. The use of fresh plant material, such as leaves harvested from the field is also a major source of microbial contamination, which is a serious problem in fermentation technology.

The patent applications WO 94/11519 and WO 97/32986 disclose methods and plants for producing degradation and conversion products in plants by the aid of a malting process. In said methods it is suggested that the enzymes are active in glyoxysomes, which catalyze the breakdown of fatty acids into acetyl-CoA. This acetyl-CoA which normally is used to make organic acids that can be exported from the glyoxysomes and used in other metabolic pathways, such as respiration and sucrose synthesis should be replaced with gene encoding enzymes in a pathway leading to polyhydroxyalkanoates useful for the production of biodegradable thermoplastics.

Even if malting process are known and have been used especially for preparing monocot plants, it is to be noted that those processes are restricted to processing starch. The methods disclosed in the patents WO 94/11519 and WO 97/32986 even if they suggest the use of the storage reserves in dicotyledons are restricted to the use of enzymes and pathways which lead sucrose and energy production and how to use these products from the respiratory pathways for resynthesis.

Even if malting is known and methods for using the respiratory pathways in plants for production of polyhydroxyalkanoates, the use of the storage reserves in dicot plants for producing proteinaceous products, such structural proteins and enzymes has not been solved.

It is to be noted that there is a great demand of proteinaceous products and because of that it is the main objective of the present invention to solve the problem of converting the storage reserves in plants into different proteinaceous products, which can be further applied for production of desired products.

The main objective of the present invention is to provide a new, more feasible, cost-effective, environmentally friendly process and production system for producing gene products, especially proteinaceous gene products in the cotyledons of transgenic dicot seeds. Another advantage of the present invention is that it provides a production system for contained use, in which the gene product can be produced in confined conditions and not in the field. This allows the present process to be carried out under almost contaminant-free conditions.

### The Summary of the Invention

The objectives of the present invention are achieved by harnessing the regulatory sequences of transient proteins accumulating during the initiation of germination for the production of desired gene products.

The characteristics of the present invention are as set out in the claims.

### The Brief Description of Drawings

Figure 1A shows a germinated transgenic seed expressing the GUS gene. The GUS gene is linked to a soybean heat shock promoter. On the right side an untransformed control seedling is shown.

Figure 1B shows a germinated transgenic seed expressing the GUS gene. The GUS gene is linked to an endopeptidase promoter.

Figure 1C shows a germinated transgenic seed expressing the GUS gene. The GUS gene is linked to a salicylate inducible promoter.

Figure 1D shows a germinated transgenic seed expressing the GUS gene. The GUS gene is linked to a 35S promoter.

Figure 2 shows the colourless substrate β-Glucuronide (100 µg/ml) which is enzymatically converted into the blue-coloured glucopyranosiduronic acid and aglycone by the GUS enzyme produced by the germinated transgenic seed.

Figure 3 depicts various germination stages of a *Brassica campestris* seed, indicated as days after the onset of germination.

Figure 4 shows a SDS-PAGE gel of germinated seeds. The samples were collected daily, starting from the dry seed. The storage proteins and Rubisco subunits are marked in the Coomassie-stained gel. After three days of germination, the amount of Rubisco protein produced per day has increased significantly as visibly demonstrated in the Figure.

Figure 5 shows a northern blot of germinated seeds using RNA probes. The samples were collected daily, starting from dry seed.

### The Detailed Description of the Invention

### Definitions

In the present invention most terms used have the same meaning as they generally have in the fields of recombinant DNA techniques, molecular biology and botany, especially in fields related to production of transgenic plants and gene products. Some terms are, however, used in a somewhat different way and are explained in more detail below.

The term "seed plants" means *Spermatophyta* the most developed plants, characterized by complex structures including fully developed organs like root, stem, leaf, flower and the vascular system. Seed plants are divided into two classes, angiosperms and gymno-sperms. Angiosperms are further divided into subclasses, i.e. monocotyledons and dicotyledons. The embryo of a mature seed of a monocot comprises only one cotyledon, which is reduced to the absorptive scutellum. In dicots the embryo has two cotyledons which serve as storage organs. The assimilates required for storage deposition in the dicot cotyledons are translocated from the mother plant through the vascular system to the seed coat and finally to the cotyledon. The seed coat is a maternal tissue and there are no symplastic connections to the embryo. The assimilates pass to the apoplasmic space and are then taken up by the embryo, redistributed symplastically and utilized in the synthesis of the reserves.

The term "dicot seed" means seed obtainable from a dicot plant comprising a versatile storage reserve including proteins, lipids and carbohydrates present in the cotyledons in contrast to the storage reserves of monocot seeds, which consist mainly of carbohydrates and only minor amounts of other compounds. In the present invention "dicot seed" means the complete seed or a fragment or a part of the seed obtainable by precrushing the seed, preferably in dry form. It is to be noted that the present invention is related to dicotyledons, monocotyledons cannot be used to achieve the objectives of the present invention.

The most preferred plant genera which can be applied for producing gene products of interest according to the process of the present invention include, but are not limited to dicot plants with high protein or oil content in seeds. Typical examples are the following genera: *Brassicaceae, Fabaceae* and *Polygonaceae.* The genus *Brassicaceae* includes for example the following species: *Brassica napus, Brassica campestris, Camelina sativa, Sinapis alba, Fabaceae* includes *Lupinus angustifolius, Phaseolus vulgaris, Glysine max, Vicia faba, Lens culinaris, Pisum sativum, Vigna mungo* and *Medicago sativa.* The genus *Polygonaceae* includes the species *Polygonum.* Also sunflower, *Helianthus annuus,* is potentially useful because of high oil contents in seeds.

The term "source-sink principle" means a production system or production entity, which can be separated into two distinct stages, the accumulation stage in which protein, carbohydrate and lipid reserves are stored into the seed and the mobilization/production stage in which the accumulated reserves normally mobilized for the initiation of growth of the plant are instead mobilized or harnessed for the production of one or more foreign gene products of interest by triggering on the expression system. The accumulation stage comprises cultivation of the transgenic plant with substantially no expression of the gene product in the field whereas the production of the gene products is restricted to the germination in confined conditions. This, is achievable by the specificity of expression systems, described in more detail elsewhere in the specification.

The term "surrounding medium" means an aqueous buffer solution either in liquid, semi-solid or solid form, which comprises substances capable of initiating, enhancing, delaying or elongating the most favourable stage of the germination. The "surrounding medium" can contain germination inducing, factors up-regulating certain processes and/or factors down-regulating certain other processes as well as external nutrients. The "surrounding medium" can also be a humid space or room.

The term "substrate" means a solid, semisolid or liquid composition or medium, which comprises at least one compound or substance, which can be transformed into another compound or substance with more desirable properties by using the composition of the present invention comprising one or more of the gene products of interest as catalyzators.

The term "seed derived composition" means the crude mixture produced by the process of the present invention obtainable from a germinating dicot seed comprising one or more *de novo* synthesized gene products expressed in the cotyledon of the germinated dicot seed including the seedling or secreted into the medium surrounding the germinating seed or seedling. The "seed derived composition" can be used as such, by placing it in contact with the substrate. Naturally the seed derived composition can be dried and/or treated with applicable down-stream processing methods as well as being isolated and/or purified before use. Alternatively, the substrate is a reagent comprising one or more compounds capable of modifying one or more of the gene products in the composition. For example substituents may be added or the folding patterns of the gene product may be modified or fragments or parts removed or added in order to get a more stable product. The "seed derived composition" can also be formulated, i.e. provided with suitable additives, such as granulation improving agents, fillers, lubricants, etc. The germinated seed-derived composition of the present invention can also be added into animal fodder to improve the digestive properties of the fodder.

The term "substantially sterile source-sink production system" means that the dicot seed can be surface sterilized with methods such as radiation or chemical sterilization. The germination can be carried cut in or on a presterilized surrounding medium, using methods applied in aseptic working methods and/or when working in sterile or super-clean conditions. The fact that the whole production can be performed in sterile conditions means that the main causes of seed being killed or not germinating, i.e. fungal or bacterial contamination of seed, can be avoided. Furthermore, the end-product is also substantially contaminant-free and not prone to degradation by contaminants having proteolytic activities. Thus, the production system is also especially suitable for producing pharmaceutical products which require high hygienic standards.

The term "initiating the germination of the transgenic dicot seed" means providing conditions favourable for germination. Optionally, it includes storing, drying, sterilizing, adding water, supplementing the seed with inducing as well as up- and/or down-regulating factors as well as external nutrition. The supplements include both physical or chemical means. External nutrition comprises for example N- and/or C-sources, vitamins, minerals, trace elements, etc. Growth factors, germination triggering factors, inducers, factors capable of up-regulating some and down-regulating other processes during the *de novo* synthesis, include for example plant hormones, such as auxin and cytokinin. The physical means includes for example a flash of light or illumination for a longer time, as well as sterilization and/or heating.

The term "expression system" means a DNA construct comprising one or more DNA sequence encoding one or more gene products of interest, operably linked with expression regulating sequences, such as enhancers, promoters and/or terminators. The expression system is preferably such that it is induced or can be induced during germination of the seed, but is substantially silent or can be silenced when the dicot plant is multiplied and cultivated in field conditions. Preferably, the expression regulating sequences comprise a promoter, which can be called a *"camera obscura*" promoter, i.e. a promoter which is triggered on or activated during germination, when the seed is kept in non-illuminated conditions in a dark room. This does not preclude the use of illumination or light. On the contrary subjecting the source-sink production system to a flash of light may increase the production of the gene product of interest by inducing and enhancing the activity of the specific promoters.

The expression systems take advantage of such expression regulating sequences which are especially active in *de novo* synthesis of transient proteins which accumulate in the cotyledon during the initiations of the germination.

Said inducible expression system comprising inducible regulatory sequences selected from the regulatory sequences of the genes encoding transient proteins which are de novo synthesized during the initiation of germination and accumulating in the cotyledons and endosperm of the plant,

Said expression systems can be prepared by selecting suitable regulatory sequences. The selection method comprises the steps of
(a) isolating total RNA from cotyledons and/or leaves of dicots;
(b) preparing cDNA from said isolated RNA by amplifying said RNA with one plant specific primer and a primer specific for a protein which is *de novo* synthesized during the initiation of germination and accumulating in the cotyledons and endosperm of the plant;
(c) collecting and plating the cDNA comprising clones;
(d) comparing the results with clones obtained from leave derived RNA; and
(e) recovering the clones comprising the cDNA showing an increased activity in germinating dicot seeds.

The term "gene product" in the present invention means proteins, polypeptides, including both structural proteins and/or enzymes. Usually, the "gene product" is a peptide, comprising at least two amino acids linked together by a peptide bond. Peptides comprising 2-10 amino acids are called oligopeptides, whereas peptides comprising more than 10 amino acids are called polypeptides. A polypeptide may be a polyamino acid chain, such as a polylysine chain consisting solely of lysine molecules, but it can also be a protein, a protein complex or a part or a fragment of the protein.

As said in the previous paragraph the term "gene product" can also mean nucleotide-based products, such as mRNA. Also included in the term are other desired biochemicals, compounds or substances, which are obtainable for example by biotransformation processes, in which one or more of the gene products present in the seed derived compositions of the present invention act as catalyzators when placed in contact with a substrate comprising the compound or substance which can be transformed to the desired product or transforms the gene product in desired manner.

Alternatively, "derivatives of the gene products" are obtainable by transformation processes, in which one or more of the original "gene products" of the present invention are modified by allowing a reagent present in the substrate to modify it. The gene products can be made more stable or more active by changing their secondary and/or tertiary structures e.g. by refolding or unfolding the amino acid chain, removing or adding fragments complexing, or adding structures or substituents to said gene products. Such "derivatives of the gene products" are also included within the term "gene products" of the present invention. Usually, the gene products produced by the process of the present invention are heterologous foreign proteins. This means that the "gene product" is not produced by the wild type plant. In other words, it is not native to the host plant. Naturally, the native seed products can be produced using the process of the present invention. It is especially useful to increase the production of certain pharmaceutically useful native homologous plant-derived products.

The term "recovering with or without down-stream processing the gene product expressed by the germinating transgenic dicot seed" means that the cotyledons including the seedlings can be recovered as such by separating them from the surrounding liquid medium with or without any down-stream processing. Alternatively, the surrounding medium is recovered as such. The storability of the "seed-derived composition" can be improved by drying, extracting, filtering, crushing, etc. Many of these means are simultaneously used for stopping the germination. Heating, drying, crushing, separating, extracting, pressing and filtering are usually applied to stop the germination before the rate of *de novo* synthesis declines and/or the concentration of the desired gene products goes down. The stopping is of importance, since the germination when allowed to continue too long, start to reuse *de novo* synthesized desired proteins for production of other substances needed by the nascent plant.

### The General Description of the Invention

The stored reserves in germinating dicot seeds, which include liberated amino acids and other biochemical substrates, comprise a renewable raw material source entity, whereas expression of the desired transgene by the transcription regulation, functioning in the cotyledon during germination and subsequent seedling growth, forms the production (sink) entity. Thus, the present invention is above all related to a production system comprising two entities, the source and the sink, i.e. two separate stages, a raw material accumulation stage and a production stage, in which the raw material source is used for *de novo* synthesis of the gene products and their derivatives or products modified by the gene product from the raw material source obtained in the first stage of the production system.

The goal of the present invention is to develop a novel process based on a source-sink production system for producing gene products in dicot seeds. The production system comprises two distinct stages. The first stage is taking advantage of the versatile renewable resources of raw material, such as protein and oil, which are accumulating in the storage reserves of the dicot plant during cultivation and which can be optionally harvested in form of seeds. The other stage comprises mobilization of the storage reserves in germinating transgenic plant for the production of one or more gene products by *de novo* synthesis of the desired gene product with or without adding supplements such as nutritional factors and providing physical and/or chemical means as well as genetical engineering for inducing germination and up- and/or down-regulating the *de novo* syntheses including transcription, expression and/or secretion.

The present invention takes in its first stage advantage of the versatile storage reserves comprising proteins, oils and/or carbohydrates, present in dicotyledons, but lacking from monocotyledons, in which the storage reserves mostly comprise two related forms of starch, amylose and amylopectin. In monocotyledons starch is mobilized by amylases during germination and subsequent growth of the nascent seedling. Resulting sucrose is used as a source of energy and carbon compounds, but the raw material for *de novo* synthesis, especially of proteins, is much more scarce than in dicots making the germinated dicot seeds into an incomparably excellent source-sink production system.

In the present invention transgenic dicot seeds are harvested from the field, transported and stored as usual. The viability of stored seeds can be maintained for long periods, usually up to several years. The temperature and seed moisture content are the major factors in determining seed viability during storage. Generally, seeds are stored at temperatures between 0-5°C. The normal moisture content of storaged seed is 4-10 %. If the moisture content is less than 20% seed respires and heat is generated. If ventilation conditions are poor, the heat generated can kill the seed. If the moisture content is over 20 % it can result in deterioration of the seed by microbial growth.

Maturation drying is the normal event in seed development during which seed passes to a metabolically quiescent state. However seed of many species may germinate without desiccation. Tomato seeds can be germinated when taken from ripening fruit and placed on water. In several other species including *Brassica campestris,* starch content rapidly decreases and the concentration of certain sugars and oligosaccharides increases during desiccation of seeds. Also certain hydrophilic proteins are expressed strongly in desiccating seed. They have the ability to attract water and they are thought to play an important role in the protection against harmful effects of desiccation. Mature dry seed may maintain germination ability and be stored from days to many years. The crude non-formulated raw material, the dicot seed, can be stably stored up to several years.

In dicot seeds, triacylglycerols are the major storage form of lipids stored in subcellular organelles as oil bodies. Seed oil content can be as high as 64% in castor bean or 48% in rape seed. Storage oil is synthesized from sucrose entering the developing seed. In the cytosol, sucrose is converted to hexosephosphate, which is translocated into the plastids or converted to glycerol-3-phosphate (glycerol backbone). In the plastids, fatty acids are synthesized starting from hexosephosphate, malate and acetate. In the endoplasmic reticulum (ER) glycerol backbone and fatty acids are esterified to give triacylglycerols.

In dicot plants the major amino acids transported in the phloem are glutamine and asparagine. In seed coat, before translocation to the cotyledons, part of the glutamine and asparagine are converted to other amino acids. In cotyledons, amino acids are used for the synthesis of storage proteins which are packed in protein bodies. Early in the seed development the cotyledon cells contain one or two large vacuoles which later become filled with storage protein and gradually fragment to form distinct and discrete smaller protein bodies. Usually dicot seed contains two or more different storage proteins. For example, *Brassica campestris* has a total protein content of 24%, containing for example 2S albumin, napin, 11S globulin and cruciferin. Of the total protein content in the seed napin represents 20% and cruciferin 60%. It is feasible to make use of the genes encoding said proteins in the present invention.

Storage proteins are generally coded by multigene families. A common feature for the luminal endoplasmic reticulum proteins of eukaryotes is a conserved carboxy-terminal tetrapeptide KDEL, which serves as the ER-retention signal and is always found at the extreme carboxyl terminus. There is no evidence that conservation of more than four terminal amino acids would be needed (Pelham, H.R.B., TIBS 15:483-486, 1990), although it has been claimed that composition of acidic residues among the terminal 20 amino acids might have some importance for efficient retention (Wandelt, C.I., et al., Plant J., 2:181-192, 1992). The suggested mechanism of retention is most likely receptor-mediated and involves binding of the KDEL-signal to the membrane receptor and fast return of the signal-receptor complex in the ER, if it is released as a vesicle (Pelham, H.R.B., TIBS 15:483-486, 1990).

Genetically modified proteins are not always found in their natural cellular compartment.

For example high-Met phaseolin expressed in transgenic tobacco is found only in the ER (endoplastic reticulum), Golgi cisternae and Golgi vesicles. Normally phaseolin accumulates in the matrix of protein storage vacuoles of developing tobacco seeds. Results indicate, that high-met phaseolin is degraded either in Golgi vesicles or just after entering the protein storage vacuoles (Hoffman, L.M., et al., Plant Mol. Biol. 11:717-730, 1988). High-level accumulation of *Vicia faba* seed storage protein vicilin with additional carboxy-terminal KDEL-sequence has been reported with tobacco and alfalfa (Wandelt, C.I., et al., Plant J. 2:181-192, 1992). Modified gene constructs fused with cauliflower mosaic virus 35S-promoter and protein are accumulated in (ER) of the leaves of transgenic plants. Accordingly, it seems feasible, that when genetically modified storage proteins are produced, retention in the ER could prevent the degradation and that a DNA construct with cauliflower mosaic virus 35S-promoter could be applied in the present invention.

Cruciferin is a hexamere of six subunits each containing one chain coded by a single gene (Rödin, J. & Rask, L., Physiol. Plant. 79:421-426, 1990). For example in *Brassica napus* cruciferin genes share approximately 60% homology between the members. Napin is the second most prominent seed protein in *Brassica napus.* Like cruciferin subunits, napin is a single-gene product composed of two different polypeptide chains linked together with disulfide bridges. Both cruciferin and napin are synthesized at the membrane of ER as precursors containing the ER-targeting signal (Ericson, M.L., et al., J. Biol. Chem., 261:14576-14581, 1986).

As mentioned earlier, both cruciferin and napin contain the ER-targeting signal sequence at their N-terminal ends. Proteins enter the ER in an unfolded state and the signal sequence is cleaved off soon after the entry. In recent years it has become clear that not all of the proteins entering the ER are further processed and packed into vacuoles. ER also contains a great number of proteins that remain in the lumen and aid the initial steps in the maturation of secretory proteins. Many of these proteins have been characterized also in mammalian systems (Pelham, H.R.B., TIBS 15:483-486, 1990), suggesting that the glycosylation of gene products, especially proteins are similar in mammals as well as in plants. In plants it has been shown that for example the auxin-binding protein is retained in the ER (Inohara, N., et al., Proc.Natl.Acad. Sci. USA 86:3564-3568, 1989).

Usually the first group of proteinases appearing during germination are SH-dependent proteinases that act on the insoluble native storage proteins. In the *Vigna mungo* synthesis of SH-dependent proteinase in the cotyledon is initiated immediately after imbibition and it increases until day 4 after which it decreases (Okamoto, T. & Minamikawa, T., J. Plant Physiol., 152(6): 675-682, 1998). The short chain peptides resulting from endopeptidase activity are cleaved from the native proteins, which in turn increases their susceptibility to other proteinases.

A second group of proteinases are inactive against native storage proteins, but hydrolyze short chain peptides to oligopeptides. At the same time also carboxypeptidases are activated and amino acids are released from peptides. This proteolysis happens in the protein body from which the oligopeptides and amino acids are released in the cytosol, where oligopeptides are degraded further into amino acids by amino- and di- or tri- peptidases. Liberated amino acids from the storage organs are further metabolized to the major transported form of amino acids, asparagine and glutamine. Storage proteins represent an amino acid source for *de novo* synthesized proteins during germination.

The second stage of the process of the present invention comprises germination in which the raw material accumulated during cultivation in the storage reserves of the dicot seed is mobilized for production of the desired gene products. The upscaling time for germination is fast and the costs low. The production volume is practically unlimited as the raw material is obtainable from renewable resources and the energy input required is obtained from the sun.

In the present invention, germination means the developmental event that begins with water-uptake by the dicot seed and increased respiration and macromolecular syntheses, storage reserve mobilization and subcellular structural changes. Physiologically, germination is a short event ending with the start of the elongation by the embryonic axis. In the present invention germination includes also the growth phase after the start of the elongation of the embryonic axis. Seed imbibes usually no more than two or three times the dry seed weight, but later in seedling development more water is needed. Germination contains numerous events like increased respiration and macromolecular syntheses and subcellular structural changes. Germination is generally completed with the radicle extension, which occurs by cell expansion without cell division. There are two types of seedlings classified according to the cotyledon fate after germination. In the hypogeal type of seedling growth, cotyledon does not raise from the soil with the developing seedling. Hypocotyl stays short but epicotyl extends and raises the first true leaves out of the soil. In the epigeal type seedling growth cotyledons are raised by expanding hypocotyl. Cotyledons usually become photosynthetically active before emerging true leaves.

When seed germinates in nature or in the field it uses the reserves of the cotyledon to start the physiological activity generally after a quiet stage as dry seed. Vital functions increase rapidly including gene expression. Significant amounts of amino acids and other biological compounds are liberated from the cotyledon storage reserves during the germination and are subsequently bound during the growth of the seedling.

Germination can also be initiated with surface sterilized seeds by incubating seeds in water or water supplemented with compounds beneficial for the production of a desired gene product. During the germination gene expression to produce the desired gene product is activated. To initiate germination, seeds are first hydrated to a moisture content of 40-50% by steeping in water tanks where temperature is adjusted according to the type of seed and the aeration is organized by compressed air. After imbibition, seeds are transferred to germinate usually in 100% moisture chambers. Typically the temperature is between 10-30°C and time ranges from 2 to 10 days, preferably 3 to 6 days, most preferably 4 to 5 days.

In the present invention the germinating dicot seed comprises a transgenic cotyledon, i.e. cotyledons of dicotyledon plants being stably transformed with a DNA sequence encoding the desired gene product. Cotyledons are a part of the embryo and they serve as a storage organ. Physiological structure and cell type diversity is relatively simple in the cotyledon. Mobilization of major stored cotyledon reserves begins after germination. In cotyledons the three major forms of storage materials, proteins, oil and carbohydrates are enzymatically converted to a transportable form during seedling growth. Storage proteins are cleaved gradually by a series of proteinases expressed in cotyledon cells. Proteinases are synthesized *de novo* by structural genes regulated with promoter region functioning in cotyledon.

During germination and subsequent growth of the nascent seedling storage lipid mobilization is initiated by lipolysis in oil bodies that yields glycerol and free fatty acids from triacylglycerol. In seed storage cell glycerol and free fatty acids are converted to sucrose in complex reactions including dozens of enzymes and at least glyoxysome and mithocon-drion organelles. Sucrose is translocated to the vacuole or embryo.

In the mobilization/production stage the accumulated protein, carbohydrate and lipid reserves are used as a source of starting material and converted into the commercially interesting products instead of the normal proteins produced to form the plant. The mobilization is started by adding to the seeds a surrounding liquid, semi-solid or solid medium, comprising water, which is supplemented with optional physical or chemical means including nutrition, germination inducing factors as well as factors capable of up-and/or down regulating certain steps in the production (transcription, expression and/or secretion). This mobilization initiates the germination and the production is going on until the emergence of green leaves.

Contamination has been known to be a serious problem in fermentation technology. A significant portion (20-25 %) of fermentation batches are contaminated in industrial scale production. Also processes that use fresh plant material like the potato starch production or corn wet milling have problems with microbial growth during the process. Fresh plant material like leaves harvested from the field is a major source of contamination which might cause a serious loss of the end-product.

The present invention describes a system that uses dry seed as raw material. There is only a limited foreign gene expression in the field, whereas the expression is mainly carried out environmentally safely in regulated conditions in a factory instead of field conditions. Dry dicotyledon seeds can be surface sterilized more easily than monocot plants because they usually have a tight, smooth seed coat unlike monocotyledon kernels. Sterilization can be performed by treatment with chemicals like hypochlorite, hydrogen peroxide or with other desinfective compounds. Since dicot seeds have a smooth surface in contrast to the wrinkled surface texture of monocot seeds, sterilization of dicot seeds is much more effective. Consequently, the production of the desired foreign or native gene product(s) can be carried out under sterile conditions, which is a great advantage especially when the target gene product is a therapeutically active protein, but also because the microbial degradation of the end product can be avoided.

The gene product may be recovered substantially contaminant-free, either as a dried plant seed derived composition, comprising one or more gene products accumulated during the germination of the transgenic dicot seed, preferably recovered at the point of maximal accumulation by breaking the germination process. The gene product can be recovered as a down-stream processable mixture of germinated dicolytedon seeds or in a storage-stable form. The composition comprises for example proteins, enzymes, peptides, hormones, growth factors, vaccines, amino acids, vitamins and/or antibiotics.

The present invention uses an engineered gene expression system to drive the synthesis to the production of the desired gene product(s) and uses the amino acids, carbon compounds and other gene products liberated during initiation of germination as a raw material source for the *de novo* synthesis of a transgene product. The natural or native gene products, e.g. proteinases, lipases, amylases, etc. formed in the germinating seeds may be used as substrates for the transgenic enzyme or to form desired biochemicals.

In the present invention the goal is to provide a transgenic plant harboring at least one expression system, comprising at least one DNA sequence encoding at least one desired gene product functionally combined with expression regulating sequences, which are induced or can be induced during germination and are substantially silent or can be silenced when a transgenic dicot plant is grown in field conditions. Promoters, active in dark (non-illuminated conditions), herein so called *camera obscura* promoters, including the more or less light independent Rubisco-promoters are useful in the expression system of the present invention.

The present invention provides possibilities to produce different desired gene products, but also other non-gene biochemicals. The transgene expressed during germination produces the first gene product, RNA, from which other RNA-products are obtainable as such or after transformation. Usually the RNA-stage is transient and the gene product recovered is a proteinaceous product. There are principally two kinds of protein products, enzymes and non-enzyme products, which often are structural proteins, vaccines, hormones, etc. The enzymes are capable of modifying an endocellular substrate obtainable from the plant, which is transformed to the desired biochemical compound. Alternatively, an extracellular substrate is added which can be transformed to give another biochemical compound. Subsequently, the protein, whether it is an enzymatically or a non- enzymatically active protein, can be placed in contact with a reagent in which case a derivative of the protein is obtained.

In the present invention heterologous foreign genes or DNA sequences are designed for optimal expression in germinating cotyledons of a dicot plant. Sequence characteristics between plant genes and heterologous genes are checked in order to express the foreign transgenic genes from heterologous sources. In plants, it is beneficial to compare the structure of these genes with known plant genes. Pioneering work in this field has been done with *Bacillus thuringiensis* endotoxin genes (*cry*). Several modifications have to be made in the original gene sequence in order to enhance expression of AT rich *cry* genes in plants.

A preferred transcriptional start sequence in plants is AACA ATG G (very conservative positions of nucleotides are shown in bold). Of the stop codons TGA, TAG and TAA, the first one has a slight preference and TAA is sparingly used in monocots. Codons in the gene sequence can be converted to appropriate ones in plants according to the codon usage tables. The converted DNA sequence should be examined for the presence of putative sites decreasing expression in plants. The putative polyadenylation signal sequences in plants usually are AATAAA, AATAAT and their variations: AACCAA, ATATAA, AATCAA, ATACTA, ATAAAA, ATGAAA, AAGCAT, ATTAAT, ATACAT and AAAATA (the most conservative of the A nucleotides are marked by bold). Putative splicing sites excluded from the gene sequence were CAN₇-₉AGTNNA. Additionally, the DNA sequence should be devoid of ATTTA sequence, which is a putative mRNA degradation element.

The transcription terminator sequences are obtainable from different genes expressed in the seed or other plant genes, for example from the *Rubisco* gene. Terminators from different bacteria, for example from *Agrobacterium* can be used as well as the *nos* gene or the *ocs* gene. The *nos* gene encodes nopaline synthase (Depicker, A., et al., (1982) J. Mol. Appl. Genet. 1:561-573 ; Shaw, C.H., et al., (1984) Nucl. Acids Res. 12:7831-7846; and An, G., et al., (1986) Mol. Gen. Genet. 203:245-250). The *ocs* gene encodes octopine synthase (Koncz, C., et al., (1983) EMBO J. 3:1597-1603. Dhaese P. et al., (1983) EMBO J. 2:419-426).

RNA polymerase II transcribes protein coding genes in plants in substantially the same way as in other eukaryotic organisms. At the general level very similar regulation elements can be found in transcription initiation regions (promoter) in eukaryotic cells. TATA box is located 25-40 nucleotides upstream of the transcription initiation site and translation initiation codon, ATG, is located 40-80 nucleotides downstream of the transcription initiation site. In mature mRNA an open reading frame follows the ATG codon and ends with one of three stop codons, UGA, UAG or UAA.

Promoters from variable sources can be used for transgene expression in plants. Many of them are from *Agrobacterium ssp.* and from plant viruses like constitutively expressed *Agrobacterium nos* promoter and cauliflower mosaic virus 35S promoter. Tissue specific promoters are useful for applications where expression in specific tissues is needed. Tissue specific promoters are usually also developmentally controlled so that they are active only at a certain developmental stage of tissue. In the present invention it is desirable that the promoter is germinating seed specific.

Rubisco (ribulose-1,5-bisphosphate carboxylase/oxygenase) represents up to 50% of total protein in germinating cotyledon. It is the most abundant plant protein that catalyzes reactions, where the CO₂ molecule condenses with ribulose-1,5-bisphoshate to form two molecules of 3-phosphoglycerate. Rubisco is located on the stromal surface of thylakoid membranes. The enzyme consists of eight large subunits, encoded by the chloroplast genome, and eight small subunit. The small subunit is encoded by a nuclear multigene family *(rbcS* genes). The small subunit is synthesized on cytoplasmic polysomes as a precursor protein. It is transported into the plastids and processed prior to the assembly of a holoenzyme. *RbcS* gene expression is tissue and developmental stage specific and is controlled by light by the phytochrome system.

In the present invention, the most preferred gene product or protein production system uses a promoter of Rubisco small subunit gene. Rubisco small subunit gene promoters can be isolated from cotyledons of *Brassica campestris* grown in dark *(camera obscura)* or non-illuminated conditions. This kind of promoter can produce foreign proteins at a very high level. Our work has shown that about 50 % of all *de novo* synthesized proteins in the dark germinating cotyledons of *B. campestris* are small and large subunit proteins of Rubisco. The mRNA level of the native *RbcS* gene transcript is as high as 500 pg per µg total RNA in germinating seeds. Maximal expression is reached about 60 hours after the start of imbibition. Similar results have been shown with mRNA levels of natural *RbcS* gene transcripts from *Brassica napus* (Fiebig, C., et al., Bot. Acta 103 (3): 258-265, 1990). Germinating seed can be illuminated with visible light for a shorter or longer time in order to increase expression. Mono-, di- or oligosaccharides, preferably sucrose or glucose can be added to repress the natural *rbcS* promoter activity. If sucrose responsive element is removed from transgene promoter it will not be repressed by sucrose. When less Rubisco enzyme is produced more free amino acids remain for the transgene expression. Also antisense technologies may be used to down-regulate endogenous *rbcS* gene expression: by expressing antisense *rbcS* gene in germinating seed.

Because Rubisco is the most abundant plant protein, it has been used as a preferred promoter in the present invention, but the expression system of the present invention is in no way restricted to the use of said promoter. An advantageous transgenic cotyledon for production of proteins comprises a promoter system for production of proteins and includes preferably the light inducible Rubisco promoter highly active in cotyledons. It is possible to produce other highly expressing Rubisco promoters by screening Rubisco cDNAs expressed in cotyledons especially in non-illuminated conditions. cDNAs may be produced by RT-PCR using oligo-T-primers and sequences from 5'-end of coding sequence of highly conserved Rubisco. In the cotyledon highly expressing cDNAs may be sequenced and variable 3' untranslated region (UTR) may be used as a the probe for detecting expression levels in plant tissues. Using cDNA sequence databases and promoters expressing essentially only in cotyledons may be isolated by using several methods. In a similar way it is possible to use cDNA information to provide other essentially seed specific and germination activated and/or inducible promoters, herein called *camera obscura*-promoters.

Many proteinases and lipases are expressed specifically in the cotyledon of the germinating seeds. One of the most well-characterized is *Vigna mungo* sulfhydryl-endopeptidase (SH-EP), which is synthesized *de novo* in the cotyledons soon after the imbibition. Generally, the amount of SH-EP mRNA increases until the third or the forth day. The SH-EP promoter region (gene bank number: EMBL X51900) is fused with GUS gene to facilitate the measurement of the expression level during germination of the dicot seed.

In addition to promoters regulated in a tissue specific manner, inducible promoters, which can be activated by various stimuli, can be used. A class of genes known as heat shock or stress genes occurs in all organisms from bacteria to man. Transcription of these genes is initiated following a stress treatment (e.g., heat shock) and translation of the transcripts produces proteins that probably protect the cell temporarily. The production of heat shock mRNAs and proteins is only a temporary phenomenon and the expression of the heat shock genes levels off, after a few hours and then declines. If the temperature is increased slowly, rather than in a single step, an organism can withstand temperatures, which would otherwise be lethal, i.e., the organism can adapt to higher temperatures. Germinated seeds can be heated to increase heat shock promoter activity. Soybean heat shock (HS) mRNA levels have been shown to increase from a barely detectable level up to 15 000-20 000 copies after two hours of treatment at 40°C. The HS promoter region described in US 5,447,858 can also be used in the expression system of the present invention as an inducible promoter. The HS promoter cloned by PCR is fused with GUS gene to measure the expression level during germination of the seed.

Several types of hydrolytic enzymes are synthesized *de novo* and functioning in germinating seeds where storage reserves are mobilized. Conversion of stored reserves to transportable form like triacylglycerol to sucrose includes several highly expressed genes like isocitrate lyase and malate dehydrogenase. Promoters from these genes can be used for transgene expression. Salicylate inducible promoter from tobacco plant, *Nicotiana tabacum,* can be cloned using PCR. It can be used to express heterologous genes in germinating seeds.

A great environmental advantage of the present invention is that the foreign gene is not expressed during cultivation in field conditions. The gene is multiplied and harvested together with the storage reserves, whereas the germination restricted expression of the foreign gene product can be carried out in confined conditions.

In principal, any commercially interesting gene product may be produced by the present invention. These gene products include enzymes, which are heterologous to the plant. In other words, they are not native to the plant species, in which they are produced. Also included are enzymes, homologous to the plants, in which they are produced. Said homologous proteins are generally overexpressed using recombinant DNA techniques. Enzymes of interest include but are not limited to hydrolases, such as proteases, β-glucanases, cellulases, hemi-cellulases, phosphatases, lipases, phospholipases, pectinases, amylases, amyloglycosidases, lysozymes, pullulanases and chitinases, peroxidases as well as lyases such as pectinolyase and isomerases, such as glucose isomerase.

It is also possible to produce structural proteins with the process and production system of the present invention. Some interesting embodiments of the present invention are for example collagen, gelatin, spidroin, silk protein, but also many other kinds of proteinaceous gene products can be produced, including enzyme inhibitors, such as the trypsine inhibitor, therapeutic peptides like interferons, insulin, neuropeptides, enkefalin, somatostatin, etc. Also other polyamino acids, such as polylysine and polyglutamate can be produced, as well as fibers, membranes, coatings, therapeutics or drugs, anti-scalants or food/feed additives and vaccines, such as LTB and growth factors, such as GM-CSF. Non-proteinaceous gene products can also be synthesized, including poly-β-hydroxy-butyrate.

As the *de novo* synthesis during germination is a rapidly passing transient situation, the germination must be broken or ended before the amount of the desired gene product starts to decline. This can be achieved by recovering the desired gene product before its concentration declines by stopping the germination either by heating, codin-N₂ (kilning) or alternatively, by crushing or excision of the germinated dicot seeds including the seedlings. If the germination is stopped by heating the desired gene product can be recovered as a crude dried product, with a good storability, which means that the enzyme can be retrieved in a crude storage stable form as well. The synthesis of the desired protein can also be stopped at its peak by crushing or excision of the germinating seed. If the germination is stopped by excision or crushing the gene product can be recovered as a crude mixture, it can be used as such or it can be down-stream processed, i.e. formulated, stored, and transported and stored. Preferably, it is used as such in e.g. in biotransformation processes. Naturally the desired gene products can be isolated and purified by conventional well known methods.

If the gene product is used for biotransformation, it is placed in contact with a substrate, i.e. a certain compound or a mixture of compounds, i.e. the gene product(s), often enzyme(s) are allowed to react with the compounds in the substrate. Either the gene product, e.g. the enzyme acts as an catalyzer and modifies the compound(s) in the substrate, or alternatively the substrate or reagent contains at least one substance or compound capable of modifying the gene product, for example its folding patterns can be modified, glycosylation or deglycosylation can be carried out or it can be provided with substituent to facilitate conjugation of the gene product with other products.

In the present invention, after the germination, in conditions where a desired gene product can be produced, the substrate is enriched with the gene product. Seed derived composition include cotyledons, hypocotyls, root and in hypogeal type plants also epicotyl and sometimes small primary leaves. The gene product can be extracted or left in the composition.

The gene product may be a protein encoded by a gene provided by the expression system of the present invention or some other biochemical compound produced by a transgenic enzyme when the native gene products stored in the seed are mobilized by germination and used as such and allowed to act on an exogenously added substrate to provide the desired (bio)transformation of the substrate.

If the gene product is extracted from the composition, it can be used either in fresh or dried form. Drying can be performed in a heated dryer at 40 - 80°C or in a lyophilizator at -20 - -80°C. The dried material can be crushed or powdered. Before extraction the composition may be mechanically disrupted and/or enzymatically treated bringing the total protein into a slurry or solution. The cellular debris may then be separated by any convenient means, such as centrifugation, sedimentation and/or filtration.

The supernatant or filtrate will normally comprise 1-40 % (w/w) desired gene product of the total protein in the medium, preferably at least about 30 % (w/w). When the desired product is not water soluble, it can for example be extracted with a convenient solvent. Alternatively, another process, which allows renaturation or solubilization and/or extraction of the product without loss of the activity of the desired product.

After isolation of the protein of interest from the aqueous medium, the gene product can be purified in conventional ways. Since the gene product will comprise a substantial portion of the total protein present in the mixture, often being the greatest percentage of any individual protein, purification is greatly simplified. Furthermore, contaminants in the product after purification are not likely to be of physiological concern for any of applications of the gene products, including therapeutic applications.

If the gene product is not extracted from the composition but left in it, it can also be recovered in fresh form or dried form. The drying can be performed as described above. The dried material can further be crushed or powdered. The fresh material can be mashed by mechanical disruption or enzymatic treatment. Dried and fresh material can be used as a source of gene product. If the gene product is an enzyme, appropriate substrates can be added into the aqueous mixture or to the dried composition or used as fresh material.

In one embodiment of the present invention, the germinated dicot seeds, cotyledons and/or seedlings comprising one or more proteins of interest, can be used as a supplement in feed products. The germinated dicot seeds can be mixed with normal non-transgenic seeds to obtain the desired concentration of the gene product of interest in an animal feed product.

Germinated seed derived mixtures or compositions can be dried and stored. The gene products can be isolated or left in the material, when for example the recombinant protein is useful in feed applications. The gene products can be used to increase protein value of animal feed or it can be for example a growth hormone or a vaccine. Also labile or toxic substances can be produced in a strictly controlled manner, which combines efficient protein production with ecological aspects and agricultural interests.

The invention is described in more detail below. The examples and experimental details are disclosed to provide an improved understanding and guidance for those skilled in the art.

### EXAMPLE 1

### GUS gene expression in germinating Brasssica campestris seeds

GUS expression is demonstrated with a histochemical assay. Four different promoters are used to regulate the GUS expression. The promoters used are Soybean heat shock promoter, *Vigna mungo* endopeptidase promoter, *Nicotiana tabacum* pr-promoter and CaMV 35S promoter. Promoter sequences are produced by PCR using plant total DNA as a template.

The promoters were linked to the GUS gene using *Nco*I enzyme. The promoter-GUS constructs were cloned in a plant transformation vector, pGPTV-hpt (Becker et al. 1992. Plant Mol. Biol. 20:1195-97). *B. campestris* was transformed according to the protocol described by Kuvshinov, V. et al. (Plant Cell Reports 18:773-777, 1999). The transgenic plants were grown in greenhouse until they produced seeds. The transgenic seeds were germinated and used for the histochemical GUS assay.

The results are described in the Figures.

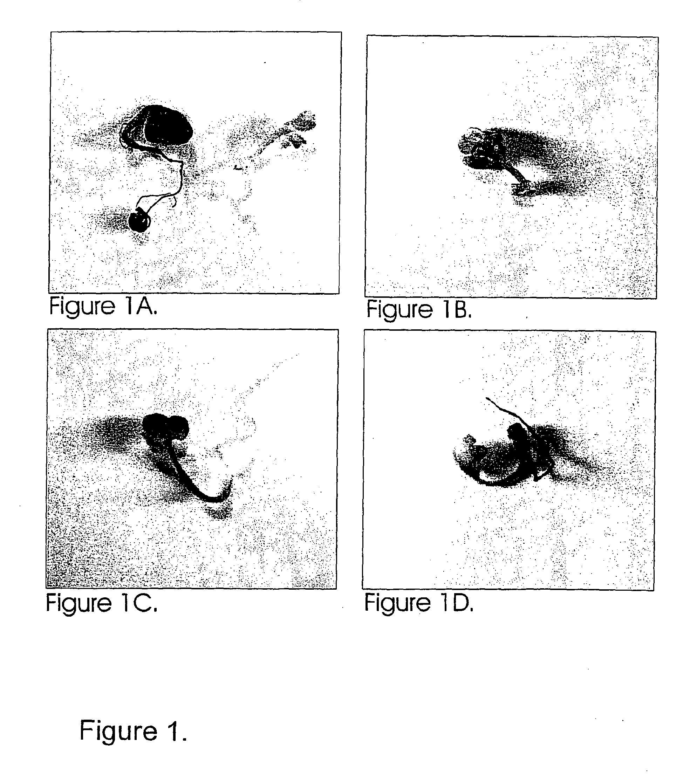

On the left side in Figure 1A a germinated seed of a transgenic *B. campestris* expressing the GUS gene is shown. The GUS gene is linked to the soybean heat shock promoter. On the right side an untransformed control seedling is shown.

In Figure 1B germinated seed of transgenic *Brassica campestris* expressing the GUS gene is shown. The GUS gene is linked to the endopeptidase promoter.

In Figure 1C the germinated seed of transgenic *Brassica campestris* expressing the GUS gene is shown. The GUS gene is linked to the salicylate inducible promoter.

In Figure 1D the germinated seed of transgenic *Brassica campestris* expressing the GUS gene is shown. The GUS gene is linked to the 35S promoter.

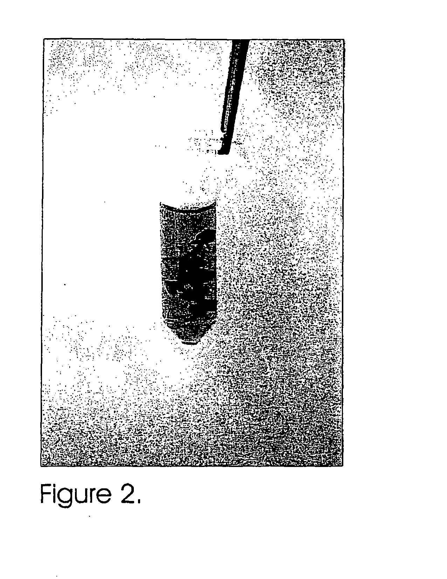

Figure 2 shows the colourless buffer solution containing the substrate β-Glucuronide (100 µg/ml) which by the action of the GUS enzyme produced by a germinated transgenic seed is enzymatically converted into the blue-coloured glucopyranosiduronic acid and aglycone.

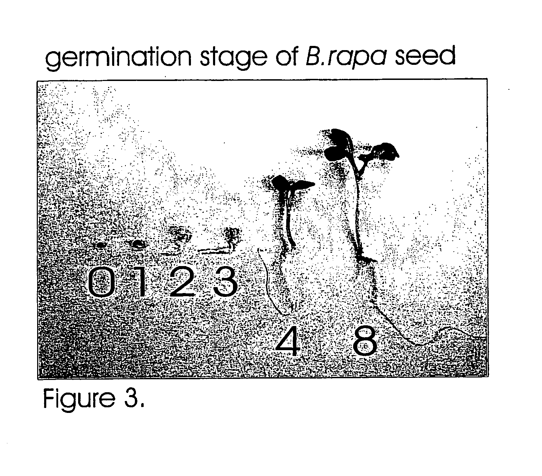

Figure 3 shows various germination stages of a *Brassica campestris* seed, indicated as days after the onset of germination. The surface sterilized seeds were germinated under *in vitro* conditions, illuminated for 16 hours at the temperature of 22°C, and under non-illuminated (dark) conditions for 8 hours at the temperature of 18°C.

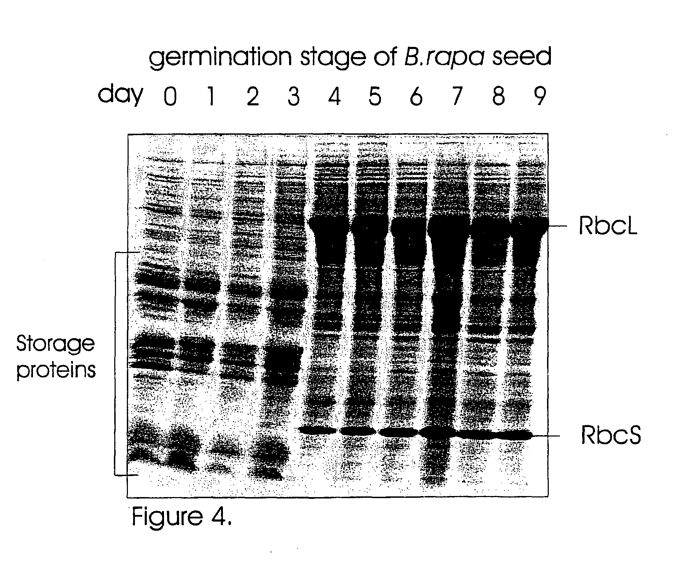

Figure 4 shows a SDS-PAGE gel of germinated seeds. The samples were collected daily, starting from the dry seed. Material was homogenized in liquid nitrogen and resuspended in 50 mM Tris, pH 8.0, in the presence of 850 mM NaCl. After centrifugation, the clear supernatant was mixed with a loading buffer. The storage proteins and Rubisco subunits are marked in the Coomassie-stained gel. After three days of germination, the amount of Rubisco protein produced per day is significant.

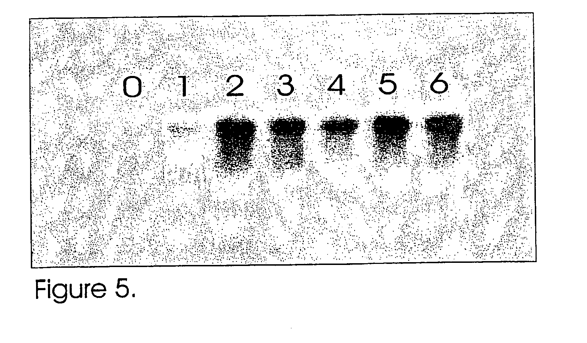

Figure 5 shows a northern blot of germinated seeds using RNA probes. The samples were collected daily, starting from dry seed. The antisense sequence probe contains 197 bp from exon 3 and 153 bp from the 3'-UTR region of rubisco small subunit cDNA (unpublished). The probe is generated from pBluescript plasmid by the T7-promoter using DIG-UTP in the reaction. Each lane was loaded with 3 µg of total RNA. RNA was extracted using the Qiagen RNeasy Plant Mini Kit. Hybridization was made with DIG Easy Hyb buffer essentially according to the manufacturer's protocol.

### EXAMPLE 2

### The preparation of novel rbcS-promoters for the expression system

### a. Isolation of a novel rbcS-promoter

*Rbc* cDNA-clones were sequenced from four days old transgenic cotyledons of *Brassica campestris.* The clones were divided into two groups with similar characteristics based on the sequence of 3'UTR region.

SDS-page analysis of *Brassica campestris* seedlings. Seeds of *Brassica campestris* were germinated in series of 1 to 7 days. Five pairs of cotyledons were ground in liquid nitrogen and resuspended in lysis-loading buffer (50 mM Tris HCl pH 8.5, 2 % SDS, 0.1% Bromophenol Blue, 10% glycerol, 2% mercaptoethanol (2-ME). Samples were boiled in a water bath for ten minutes and analysed in 15 % SDS-PAGE gel according to the Laemmli (1970). The *Rbc* gene expression was estimated to initiate 4 days after imbibition.

### b. RNA-purification from Brassica campestris cotyledons.

RNA was purified from 4 days old cotyledons by using Qiagen RNeasy Kit.

### c. cDNA-synthesis was carried out from total RNA of Brassica campestris cotyledons.

2 µg of total RNA and 10 ng of *notI-*d(T)18 primer in 16 µl of water was incubated for 5 minutes at a temperature of 70°C. The mixture was transferred on ice and 5µl of 5 x reaction buffer, 2.5 µl of 5mM dNTP, 20 U of ribonuclease inhibitor and 200 U of M-MLV reverse transcriptase was added. The reaction mixture was incubated at a temperature of 42°C for 1 hour.

### d. PCR-amplification of cDNA clones expressed in cotyledon cells.

PCR was initially optimized by using series of twelve primers consisting of thymine nucleotides and a changing dinucleotide anchor as a 3'-primer and a 49-mer oligonucleotide from the starting region of the *Brassica napus rbc* gene (Gene bank accession number g17849) exon no III as a 5'-primer. Initial optimization showed that only GCT₁₁ as a 3'-primer resulted in a PCR-product. In order to clone this PCR-product, a 49-mer oligonucleotide was constructed with *Not*I-restriction site. The primers are listed in Table 1.

**Table 1. Primers used for PCR-isolation of rubisco cDNA-clones.**
3'primers for initial screening
1: 5'TTTTTTTTTTTCA3' (SEQ.ID:1:)
2: 5'TTTTTTTTTTTCT3' (SEQ.ID:2:)
3: 5'TTTTTTTTTTTCC3' (SEQ.ID:3:)
4: 5'TTTTTTTTTTTCG3' (SEQ.ID:4:)
5: 5'TTTTTTTTTTTGA3' (SEQ.ID:5:)
6: 5'TTTTTTTTTTTGT3' (SEQ.ID:6:)
7: 5'TTTTTTTTTTTGC3' (SEQ.ID:7:)
8: 5'TTTTTTTTTTTGG3' (SEQ.ID:8:)
9: 5'TTTTTTTTTTTAA3' (SEQ.ID:9:)
10: 5'TTTTTTTTTTTAT3' (SEQ.ID:10:)
11: 5'TTTTTTTTTTTAC3' (SEQ.ID:11:)
12: 5'TTTTTTTTTTTAG3' (SEQ.ID:12:)
5'-primer (SEQ.ID:13:):
5'CGCGGATCCCACGGGTTTGTTTACCGTGAGCACGGAAGCACCCCCGGAT3'
3'-primer for cloning of the rbc cDNA-clone (SEQ.ID:14:):
5' AAGGAAAAAAGCGGCCGCAATTTTTTTTTTTTTTTTTTTTTTTTTTTGC3'

For the cloning of the *rbc* cDNA-fragment, PCR-amplification was carried out in using 1 µl of cDNA-synthesis reaction mixture as a template, 100 nM 5'-primer, 100 nM 3'-primer, 100 µM dNTP and 1.25 U of *Pfu* DNA-polymerase/25 µl of reaction volume. The obtained PCR-fragment was cloned into the *Not*I and *Bam*HI-sites of a pUK21-vector. Seven individual clones were sequenced. According to the sequence, clones were divided into two groups containing 3 similar respectively 4 similar representatives. One representative from both groups was chosen for further studies. The sequences were named *utr2* (SEQ.ID:15:) and *utr*8 (SEQ.ID:16:)

### e. Cloning of Brassica campestris rubisco small subunit promoter Type I (65A)

The 2.8 kb fragment carrying the promoter and the gene corresponding to UTR 65A (SEQ.ID:24:) was amplified using bnrb1 (SEQ.ID:17:) (5'-GAATTCTAACGACCCTTTTCCG-3') which is complementary to *B. napus* rubisco small subunit gene as 5'-primer and UTR2 (SEQ.ID:18:) (5'-GGCCACACTTGACAATCCGATATAACATGCCTCA-3') which is specific to UTR 65A (SEQ.ID:24:) as 3'-primer. The amplification was done with *Pfx* Platinum DNA polymerase (LifeTechnologies) according to manufacturer s recommendations. The promoter regions were amplified using the obtained fragment as a template. Bnrb3 (SEQ.ID:19:) (5'-AAAAAGCTTCTAGACCCTTTTCCGTCATAAGTTTTATA-3') which is complementary to *B. napus* rubisco small subunit gene was used as 5'-primer and RbSiB (SEQ.ID:20:) and RBNCO (SEQ.ID:21:) as 3'-primers in separate reactions. RbSiB (5'-CAGGTCTCCCATGCAGCTAACTCTTCCTCCGTTGCT-3') has 3' end complementary to the end of putative chloroplast targeting signal peptide of *B. napus* rubisco small subunit gene and 5'-end which carries *Eco*311 site which after cutting leaves ATG-containing Ncol site-compatible end for cloning into plant expression vectors. RBNCO (SEQ.ID:21:) (5'-GAGGAAGCCATGGCTACTTCTT-3') is compatible to the start of the coding region of the *B. napus* rubisco small subunit gene except two-nucleotide change, which creates a *Nco*I site around the ATG codon.

### f. Evaluation of mRNA pool coding small subunit of ribulose-1,5-bisphosphate carboxylase in Brassica campestris leaves and seedlings.

Total RNA was isolated from mature leaves and four day old seedlings using RNeasy kit (Qiagen). Messenger RNA was further purified using Oligo (dT) Cellulose column according manufacturer's recommendation (New England Biolabs). Oligo T primer (SEQ.ID:22:) (CUACUACUACUAGCGGCCGCTTTTTTTTTTTTTTTTTTTTTTTTT VN) and Moloney Murine Leukaemia Virus Reverse Transcriptase (Promega) were used to synthesize total cDNA pool. Rubisco small subunit cDNAs were amplified using two cycles of PCR (1 min 94°C, 1 min 55°C 1 min 72°C). PCR primers; oligo T primer and primer which recognizes the end of the last exon of rubisco small subunit (SEQ.ID:23:) (CAUCAUCAUCAUTCGACGATCATCGGATTCGACA). The PCR product was digested with Uracil DNA Glycosylase and cloned to pAMP1 vector (CloneAMP pAMP System LIFE TECHNOLOGIES). A total of 102 clones was analysed by DNA sequencing. 42 clones were from germinating seedlings and 60 clones from mature leaves. Three different types (Type I, II and III) of mRNA were found. The sequences and percentage of the distribution of different types of sequences in germinating seedlings and mature leafs are presented in Table 2.

**Table 2. The sequences and percentage of distribution of different types of mRNA.**

| SEQUENCE | Germinating seedling | Mature Leaf |
|---|---|---|
| Type I | 29 % | 26 % |
| Type II | 15 % | 30 % |
| Type III | 56 % | 44 % |

The sequences of three different types of mRNA are as follows.

### g. Cloning of the most abundant Brassica campestris rubisco small subunit gene

One of the *B. campestris* rubisco small subunit genes represents 56 % of the transcripts according to quantitative sequencing analysis of 102 cloned rubisco small subunit UTR-clones. This clone was named UTR56 (Type III, SEQ.ID:26:). In order to clone the corresponding gene and its upstream region four primers were designed to be used in two-step PCR. In the first PCR, primer bnrb4 (SEQ.ID:27:) (5'-GGCCATGAATTCTAACGACCCTTTTCC GTCATAAAAGT-3') which is a part of *B. napus* rubisco small subunit gene (accession number x61097) was used as 5'-primer. The primer 56r6 (SEQ.ID:28:) (5'-CGCGATATAGAAATTACATGTTCATAAAATCAGACATTTTAC-3') specific to UTR56 (SEQ.ID:26:) was used as 3'-primer. The composition of the first PCR was: 75 mM Tris, pH 8.0 at 25°C, 20 mM (NH₄)SO₄, 0.01 % Tween 20, 2mM MgCl₂, 200 µM dNTPs, 0.2 µM primers, 0.025 units/µl *Taq* DNA polymerase, 0.0016 units/µl *Pfu* DNA polymerase and 100 pg/µl *B. campestris* DNA. The program was: 94° C, 4 minutes, 30 X [(94°C, 30 s), (38°C, 30 s), (72°C, 3 min)]. The expected 2.8 kb fragment was purified from agarose gel. Because there was no certain sequence information, the fragment was reamplified using bnrb5 (SEQ.ID:29:) (5'-GAGGTACCCGCGGCCGC GAATTCTAACGACCC TTTTCCGTCATAAAAG-3') which has 3'-end complementary to bnrb4 (SEQ.ID:27:) and 5'-end extension which carries *Not*I 8-base cut site and 56r7 (SEQ.ID:30:) (5'-GAGAATTCGGCGCGCCATAGAAATTACATGTTCATAAAATC AGACA-3') which has 3'-end complementary to 56r6 (SEQ.ID:28:) and 5'-end extension which carries *Asc*I 8-base cut site. The PCR composition was similar as in the first PCR with following modifications: 25 pg/µl of gel-purified fragment from the first PCR was used as a template and only 7 cycles were performed.

The obtained product was precipitated, cut with *Asc*I and *Not*I restriction enzymes and gel-purified. The fragment was cloned into a modified pNEB193, pAN (*Pme*I site was changed to *Not*I site) which was opened with *Asc*I and *Not*I*,* treated with Shrimp alkaline phosphatase, and after heat inactivation of the enzymes gel-purified. Four of the obtained clones were sequenced in order to verify that they carry the UTR56 sequence.

The promoter region was amplified using bnrb5 (SEQ.ID:29:) as 5'-primer and as 3'-primers RbSiB (SEQ.ID:20) and RBNCO (SEQ.ID:21:) (see above). The PCR conditions were similar to those which were used to amplify the whole gene with following modifications: 5 ng/µl of plasmid DNA carrying the clones (linearized with *Asc*I) were used as templates, only 10 cycles was performed.

### Example 3.

### Comparison of different constitutive and inducible promoters for the overexpression of transgene (GUS) in tobacco and Brassica campestris

Germinating seedlings were frozen with liquid nitrogen and grinded. Proteins were extracted with phosphate-EDTA buffer. Samples were centrifuged and supernatants were analysed using β-glucuronidase activity detection kit (Sigma). Protein concentration of samples were analysed with Protein assay reagent (Biorad) using BSA as a standard.

**Table 3. The specific activities of GUS with different constitutive and inducible promoters in tobacco and Brassica campestris.**

| PLANT | PROMOTER | SPECIFIC ACTIVITY (nmol MU/min/mg of soluble protein) |
|---|---|---|
| tobacco | *B.* rubisco type I | 7 |
| *B. campestris* | Heat shock | 44 |
| *B. campestris* | Heat shock amylase | 5 |
| *B. campestris* | 35S | 28 |

When following the promoter activity in germinating seed during germination in a series of seven days an exponential increase in specific activity for example in rubisco promoter was detected. The specific activity of rubisco promoter was 0.25 on the 4th day, 0.5 on the 5th day, 1.6 on the 6th day and 7 on the 7th day.

### Analysis of chloroplast development inhibitors for rubisco protein and mRNA levels in germinating seeds

Analysis of germinating natural *B. campestris* seedlings have shown that rubisco small subunit protein get highest concentration three days after initiation of germination and rubisco small subunit mRNA one day earlier. We have found that by adding streptomycin 100 mg/l into the growth media 48 hour after initiation of germination it is possible to down regulate rubisco protein synthesis but mRNA level remain constant. Because rubisco protein is major protein in certain types of germinating cotyledons it is beneficial to down regulate its synthesis. Seeds were germinated in aerated water supplemented streptomycin. Samples were collected 12 h intervals. Protein levels were analysed from SDS-PAGE gel and mRNA levels by RNA dot blot analysis.

## Claims

1. A process based on a source-sink principle for converting storage reserves in transgenic *Brassicaceae* seeds to a composition comprising one or more desired heterologous proteins,
**characterized in that** said process comprises the steps of:
(a) cultivating a transgenic *Brassicaceae* plant having an expression system inducible during the seed germination, in order to provide a storable internal source of starting material, which can be harvested as transgenic seeds, wherein said inducible expression system comprises one or more DNA sequences encoding one or more heterologous proteins of interest, said DNA sequences being operably linked to regulatory sequences providing high expression levels in cotyledons of genninating *Brassicaceae* seeds;
(b) harvesting seeds of the transgenic *Brassicaceae* plant;
(c) germinating the transgenic *Brassicaceae* seeds comprising one or more expression systems integrated into the plant-genome in contact with a surrounding medium, during which germination the internal source of the starting material is mobilized and the expression system is induced;
(d) harnessing the expression system integrated in the plant genome for *de novo* synthesis of one or more of the desired heterologous proteins from the internal source of starting material mobilized in step (c);
(e) stopping the germination before *de novo* synthesis declines; and
(f) recovering the composition comprising germinating *Brassicaceae* seeds or seedlings.

2. The process according to claims 1, **characterized in that** the regulatory sequence is a promoter of a gene encoding a Rubisco protein.

3. A process based on a source-sink principle for converting storage reserves in transgenic dicotyledonous seeds to a composition comprising one or more desired heterologous proteins,
**characterized in that** said process comprises the steps of:
(a) cultivating a transgenic dicotyledonous plant having an expression system inducible during the seed germination in order to provide a storable internal source of starting material, which can be harvested as transgenic dicotyledonous seeds, wherein said inducible expression system comprises one or more DNA sequences encoding one or more heterologous proteins of interest, said DNA sequences being operably linked to a promoter of a gene encoding a Rubisco protein providing high expression levels in cotyledons of germinating dicotyledonous seeds;
(b) harvesting seeds of the transgenic dicotyledonous plant;
(c) germinating the transgenic dicotyledonous seeds comprising one or more expression systems integrated into the plant-genome in contact with a surrounding medium, during which germination the internal source of the starting material is mobilized and the expression system is induced;
(d) harnessing the expression system integrated in the plant genome for *de novo* synthesis of one or more of the desired heterologous proteins from the internal source of starting material mobilized in step (c);
(e) stopping the germination before *de novo* synthesis declines; and
(f) recovering the composition comprising germinating dicotyledonous seeds or seedlings.

4. The process according to claim 3, **characterized in that** the transgenic dicotyledonous plant derives from *Brassicaceae.*

5. The process according to any of claims 1 to 4, **characterized in that** the transgenic plant is *Camelina sativa.*

6. The process according to any of claims 1to 5, **characterized in that** the harnessing for *de novo* synthesis is provided by down-regulating the endogenous gene expression leaving more free amino acids for the transgene expression in the germinating seed or seedling.

7. The process according to claim 6, **characterized in that** the down-regulation of the gene is provided by repression or antisense technologies.

8. The process according to claim 6 or 7, **characterized in that** the down-regulated gene encodes a Rubisco protein.

9. The process according to any of claims 1 to 8, **characterized in that** the composition comprising at least one heterologous protein is placed in contact with a substrate comprising at least one compound which can be transformed by a biochemical reaction catalyzed by one or more of said heterologous proteins.

10. The process according to any of claims 1 to 9, **characterized in that** the composition comprising at least one heterologous protein is placed in contact with a reagent comprising at least one compound capable of modifying one or more of the heterologous proteins.

11. The process according to any of claims 1 to 10, **characterized in that** the source of starting material comprising the transgenic seed is optionally sterilized and the germination is performed under substantially sterile conditions providing a substantially sterile source-sink production system.

12. The process according to any of claims 1 to 11, **characterized in that** the promoter is substantially silent or can be silenced when cultivating the transgenic plant in field conditions.

13. The process according to any of claims 1 to 12, **characterized in that** the surrounding medium is an optionally buffered aqueous solution.

14. The process according to any of claims 1 to 13, **characterized in that** the source-sink production system is supplemented with at least one physical or chemical means for inducing germination.

15. The process according to any of claims 1 to 14, **characterized in that** the source-sink production system is supplemented with at least one external nutrient.

16. The process according to any of claims 1 to 15, **characterized in that** the source-sink production system is supplemented with at least one means for up-regulating the *de novo* synthesis of the desired heterologous protein(s).

17. The process according to any of claims 1 to 16, **characterized, in that** the source-sink production system is supplemented with at least one means for down-regulating the *de novo* synthesis of endogenous gene product(s) normally produced during germination.

18. The process according to any of claim 1 to 17, **characterized in that** the germination is optionally stopped by at least one means comprising heating, drying, crushing, separating, extracting, pressing and filtering and subsequently recovering with or without down-stream processing methods at least one of the heterologous proteins.

19. The process according to any of claims 1 to 18 for producing a seed derived composition, **characterized in that** the composition is substantially contaminant-free composition comprising one or more heterologous proteins synthesized in the cotyledons of a transgenic seed or seedling.

20. The process according to claim 19, **characterized in that** the composition further comprises a surrounding medium.

21. The process according to claim 19 or 20, **characterized in that** the composition is provided in dried or down-stream processed form.

22. The process according to any of claims 19 to 21, **characterized in that** the composition further comprises one or more applicable, formulating ingredients.

## Patentansprüche

1. Verfahren basierend auf einem Quelle-Senk-Prinzip zur Umwandlung von Speicherreserven in transgenen *Brassicaceae*-Samen in eine Zusammensetzung, die ein oder mehrere gewünschte heterologe Protein(e) umfasst, **dadurch gekennzeichnet, dass** dieses Verfahren die Schritte umfasst:
(a) Kultivieren einer transgenen *Brassicaceae*-Pflanze mit einem Expressionssystem, das während der Samenkeimung induzierbar ist, um eine speicherbare interne Quelle von Ausgangsmaterial bereitzustellen, welche als transgene Samen geerntet werden können, wobei dieses induzierbare Expressionssystem eine oder mehrere DNA-Sequenz(en) umfasst, die ein oder mehrere heterologe Protein(e) von Interesse codieren, wobei diese DNA-Sequenz(en) operativ mit regulatorischen Sequenzen verbunden ist/sind, die hohe Expressionsniveaus in Kotyledonen keimender *Brassicaceae-Samen* bereitstellen;
(b) Ernten der Samen der transgenen *Brassicaceae*-Pflanze;
(c) Keimen der transgenen *Brassicaceae*-Samen umfassend ein oder mehrere Expressionssystem(e), das/die in das Pflanzengenom in Kontakt mit einem umgebenden Medium integriert ist/sind, wobei während der Keimung die interne Quelle des Ausgangsmaterials mobilisiert wird und das Expressionssystem induziert wird;
(d) Nutzbarmachen des in das Pflanzengenom integrierten Expressionssystems für die *de novo*-Synthese eines oder mehrerer gewünschter heterologer Proteins/Proteine aus der internen Quelle des Ausgangsmaterials, welches in Schritt (c) mobilisiert wird;
(e) Stoppen der Keimung bevor die *de novo*-Synthese abnimmt; und
(f) Gewinnen der Zusammensetzung, die keimende *Brassicaceae-Samen* oder Keimlinge umfasst.

2. Verfahren gemäß Anspruch 1, **dadurch gekennzeichnet, dass** die regulatorische Sequenz ein Promotor eines Gens ist, das ein Rubiscoprotein codiert.

3. Verfahren basierend auf einem Quelle-Senk-Prinzip zur Umwandlung von Speicherreserven in transgenen dikotyledonen Samen in eine Zusammensetzung, die ein oder mehrere gewünschte heterologe Protein(e) umfasst, **dadurch gekennzeichnet, dass** dieses Verfahren die Schritte umfasst:
(a) Kultivieren einer transgenen dikotyledonen Pflanze mit einem Expressionssystem, welches während der Samenkeimung induzierbar ist, um eine speicherbare interne Quelle von Ausgangsmaterial bereitzustellen, welche als transgene dikotyledone Samen geerntet werden kann, wobei dieses induzierbare Expressionssystem eine oder mehrere DNA-Sequenz(en) umfasst, welche ein oder mehrere heterologisch Protein(e) von Interesse codiert/codieren, wobei die DNA-Sequenz(en) operativ mit einem Promotor eines Gens verbunden ist/sind, der ein Rubiscoprotein codiert, welches erhöhte Expressionsniveaus in Kotyledonen keimender dikotyledoner Samen bereitstellt;
(b) Ernten von Samen der transgenen dikotyledonen Pflanze;
(c) Keimen der transgenen dikotyledonen Samen umfassend ein oder mehrere Expressionssystem(e), das/die in das Pflanzengenom integriert ist/sind in Kontakt mit einem umgebenden Medium, wobei während der Keimung die interne Quelle des Ausgangsmaterials mobilisiert wird und das Expressionssystem induziert wird;
(d) Nutzbarmachen des Expressionssystems, welches in das Pflanzengenom integriert ist, für die *de novo*-Synthese eines oder mehrerer gewünschten heterologen Proteins/Proteine aus der internen Quelle des Ausgangsmaterials, welches in Schritt (c) mobilisiert wurde;
(e) Stoppen der Keimung bevor die *de novo*-Synthese abnimmt; und
(f) Gewinnen der Zusammensetzung, die keimende dikotyledone Samen oder Keimlinge umfasst.

4. Verfahren gemäß Anspruch 3, **dadurch gekennzeichnet, dass** die transgene dikotyledone Pflanze von *Brassicaceae* abstammt.

5. Verfahren gemäß einem der Ansprüche 1 bis 4, **dadurch gekennzeichnet, dass** die transgene Pflanze *Camelina sativa* ist.

6. Verfahren gemäß einem der Ansprüche 1 bis 5, **dadurch gekennzeichnet, dass** das Nutzbarmachen zur *de novo*-Synthese durch Herunterregulierung der endogenen Genexpression ermöglicht wird, so dass mehr freie Aminosäuren für die Transgenexpression in dem keimenden Samen oder Keimling bleiben.

7. Verfahren gemäß Anspruch 6, **dadurch gekennzeichnet, dass** die Herunterregulierung des Gens durch Repression oder Antisense-Technologien erfolgt.

8. Verfahren gemäß Anspruch 6 oder 7, **dadurch gekennzeichnet, dass** das herunterregulierte Gen ein Rubiscoprotein codiert.

9. Verfahren gemäß einem der Ansprüche 1 bis 8, **dadurch gekennzeichnet, dass** die Zusammensetzung, die mindestens ein heterologes Protein umfasst, in Kontakt gebracht wird mit einem Substrat, das mindestens eine Verbindung umfasst, welche durch eine biochemische Reaktion umgewandelt werden kann, welche durch eines oder mehrere dieser heterologen Proteine katalysiert wird.

10. Verfahren gemäß einem der Ansprüche 1 bis 9, **dadurch gekennzeichnet, dass** die Zusammensetzung, die mindestens ein heterologes Protein umfasst, in Kontakt gebracht wird mit einem Reagens, welches mindestens eine Verbindung umfasst, die in der Lage ist, ein oder mehrere der heterologen Protein(e) zu modifizieren.

11. Verfahren gemäß einem der Ansprüche 1 bis 10, **dadurch gekennzeichnet, dass** die Quelle des Ausgangsmaterials, welches die transgenen Samen umfasst, gegebenenfalls sterilisiert wird und die Keimung unter im wesentlichen sterilen Bedingungen durchgeführt wird, wodurch ein im wesentlichen steriles Quelle-Senk-Produktionsverfahren zur Verfügung gestellt wird.

12. Verfahren gemäß einem der Ansprüche 1 bis 11, **dadurch gekennzeichnet, dass** der Promotor im wesentlichen stumm ist, oder dass er während der Kultivierung der transgenen Pflanze unter Feldbedingungen stumm gemacht werden kann.

13. Verfahren gemäß einem der Ansprüche 1 bis 12, **dadurch gekennzeichnet, dass** das umgebende Medium eine gegebenenfalls gepufferte wässrige Lösung ist.

14. Verfahren gemäß einem der Ansprüche 1 bis 13, **dadurch gekennzeichnet, dass** das Quelle-Senk-Produktionssystem mit mindestens einem physikalischen oder chemischen Mittel zur Induzierung der Keimung ergänzt wird.

15. Verfahren gemäß einem der Ansprüche 1 bis 14, **dadurch gekennzeichnet, dass** das Quelle-Senk-Produktionssystem mit mindestens einem externen Nährstoff ergänzt wird.

16. Verfahren gemäß einem der Ansprüche 1 bis 15, **dadurch gekennzeichnet, dass** das Quelle-Senk-Produktionssystem mit mindestens einem Mittel zur Hochregulierung der *de novo*-Synthese des/der gewünschten heterologen Proteins/Proteine ergänzt ist.

17. Verfahren gemäß einem der Ansprüche 1 bis 16, **dadurch gekennzeichnet, dass** das Quelle-Senk-Produktionssystem mit mindestens einem Mittel zur Herunterregulierung der *de novo*-Synthese von endogenem/n Genprodukt(en) ergänzt wird, das/die normalerweise während der Keimung produziert wird/werden.

18. Verfahren gemäß einem der Ansprüche 1 bis 17, **dadurch gekennzeichnet, dass** die Keimung gegebenenfalls durch mindestens ein Mittel gestoppt wird, umfassend Erhitzen, Trocknen, Zerdrücken, Trennen, Extrahieren, Pressen und Filtrieren und anschließend Gewinnen mit oder ohne nachfolgende Verarbeitungsverfahren von mindestens einem der heterologen Proteine.

19. Verfahren nach einem der Ansprüche 1 bis 18 zum Produzieren einer aus Samen stammenden Zusammensetzung, **dadurch gekennzeichnet, dass** die Zusammensetzung im wesentlichen frei von Verunreinigungen ist und ein oder mehrere in den Kotyledonen eines transgenen Samens oder Keimlings synthetisierte heterologe Proteine umfasst.

20. Verfahren gemäß Anspruch 19, **dadurch gekennzeichnet, dass** die Zusammensetzung weiter ein umgebendes Medium umfasst.

21. Verfahren gemäß Anspruch 19 oder 20, **dadurch gekennzeichnet, dass** die Zusammensetzung in getrockneter oder durch Downstream-Verarbeitung erhaltener Form bereitgestellt wird.

22. Verfahren gemäß einem der Ansprüche 19 bis 21, **dadurch gekennzeichnet, dass** die Zusammensetzung weiter einen oder mehrere anwendbare Formulierungsbestandteile umfasst.

## Revendications

1. Procédé à base d'un principe "source-sink" pour convertir des réserves de stockage dans des graines de *Brassicaceae* transgéniques en une composition comprenant une ou plusieurs protéines hétérologues souhaitées, **caractérisé en ce que** ledit procédé comprend les étapes consistant à:
(a) cultiver une plante *Brassicaceae* transgénique ayant un système d'expression inductible pendant la germination des graines afin de produire une source interne stockable de matière première, que l'on peut récolter sous la forme de graines transgéniques, où ledit système d'expression inductible comprend une ou plusieurs séquences d'ADN codant pour une ou plusieurs protéines hétérologues d'intérêt, lesdites séquences d'ADN étant en liaison fonctionnelle avec des séquences régulatrices produisant des niveaux élevés d'expression dans les cotylédons des graines de *Brassicaceae* en germination;
(b) récolter les graines de la plante *Brassicaceae* transgénique;
(c) provoquer la germination les graines de *Brassicaceae* transgéniques comprenant un ou plusieurs systèmes d'expression intégrés dans le génome de la plante en contact avec un milieu environnant, germination pendant laquelle la source interne de la matière première est mobilisée et le système d'expression est induit;
(d) atteler le système d'expression intégré dans le génome de la plante pour obtenir une synthèse de *novo* d'une ou plusieurs des protéines hétérologues souhaitées de la source interne de la matière première mobilisée à l'étape (c);
(e) arrêter la germination avant que le déclin de la synthèse de *novo*; et
(f) récupérer la composition comprenant les graines de *Brassicaceae* en germination ou les jeunes plants.

2. Procédé selon la revendication 1, **caractérisé en ce que** la séquence régulatrice est un promoteur d'un gène codant pour une protéine Rubisco.

3. Procédé à base d'un principe "source-sink" pour convertir des réserves de stockage dans des graines dicotylédones transgéniques en une composition comprenant une ou plusieurs protéines hétérologues souhaitées, **caractérisé en ce que** ledit procédé comprend les étapes consistant à:
(a) cultiver une plante dicotylédone transgénique ayant un système d'expression inductible pendant la germination des graines afin de produire une source interne stockable de matière première, que l'on peut récolter sous la forme de graines dicotylédones transgéniques, où ledit système d'expression inductible comprend une ou plusieurs séquences d'ADN codant pour une ou plusieurs protéines hétérologues d'intérêt, lesdites séquences d'ADN étant en liaison fonctionnelle avec un promoteur d'un gène codant pour une protéine Rubisco produisant des niveaux élevés d'expression dans les cotylédons des graines dicotylédones en germination;
(b) récolter les graines de la plante dicotylédone transgénique;
(c) provoquer la germination des graines dicotylédones transgéniques comprenant un ou plusieurs systèmes d'expression intégrés dans le génome de la plante en contact avec un milieu environnant, germination pendant laquelle la source interne de la matière première est mobilisée et le système d'expression est induit;
(d) atteler le système d'expression intégré dans le génome de la plante pour obtenir une synthèse de *novo* d'une ou plusieurs des protéines hétérologues souhaitées de la source interne de la matière première mobilisée à l'étape (c);
(e) arrêter la germination avant que le déclin de la synthèse de *novo*; et
(f) récupérer la composition comprenant les graines dicotylédones en germination ou les jeunes plants.

4. Procédé selon la revendication 3, **caractérisé en ce que** la plante dicotylédone transgénique est dérivée de *Brassicaceae.*

5. Procédé selon l'une quelconque des revendications 1 à 4, **caractérisé en ce que** la plante transgénique est *Camelina sativa.*

6. Procédé selon l'une quelconque des revendications 1 à 5, **caractérisé en ce que** l'attelage pour une synthèse de novo est formé par une régulation vers le bas de l'expression du gène endogène laissant plus d'acides aminés libres pour l'expression du transgène dans la graine en germination ou le jeune plant.

7. Procédé selon la revendication 6, **caractérisé en ce que** la régulation vers le bas du gène est obtenue par des technologies de répression ou antisens.

8. Procédé selon la revendication 6 ou 7, **caractérisé en ce que** le gène régulé vers le bas code pour une protéine Rubisco.

9. Procédé selon l'une quelconque des revendications 1 à 8, **caractérisé en ce que** la composition comprenant au moins une protéine hétérologue est placée en contact avec un substrat comprenant au moins un composé qui peut être transformé par une réaction biochimique catalysée par une ou plusieurs desdites protéines hétérologues.

10. Procédé selon l'une quelconque des revendications 1 à 9, **caractérisé en ce que** la composition comprenant au moins une protéine hétérologue est placée en contact avec un réactif comprenant au moins un composé capable de modifier une ou plusieurs des protéines hétérologues.

11. Procédé selon l'une quelconque des revendications 1 à 10, **caractérisé en ce que** la source de matière première comprenant la graine transgénique est facultativement stérilisée et la germination est accomplie en conditions sensiblement stériles formant un système de production source-sink sensiblement stérile.

12. Procédé selon l'une quelconque des revendications 1 à 11, **caractérisé en ce que** le promoteur est sensiblement silencieux ou peut être mis sous silence lors de la culture de la plante transgénique en conditions champêtres.

13. Procédé selon l'une quelconque des revendications 1 à 12, **caractérisé en ce que** le milieu environnant est une solution aqueuse facultativement tamponnée.

14. Procédé selon l'une quelconque des revendications 1 à 13, **caractérisé en ce que** le système de production source-sink est complété d'au moins un moyen physique ou chimique pour induire la germination.

15. Procédé selon l'une quelconque des revendications 1 à 14, **caractérisé en ce que** le système de production source-sink est complété avec au moins un aliment externe.

16. Procédé selon l'une quelconque des revendications 1 à 15, **caractérisé en ce que** le système de production source-sink est complété d'au moins un moyen pour réguler vers le haut la synthèse *de novo* de la ou des protéines hétérologues souhaitées.

17. Procédé selon l'une quelconque des revendications 1 à 16, **caractérisé en ce que** le système de production source-sink est complété d'au moins un moyen pour réguler vers le bas la synthèse de *novo* du ou des produits de gène endogène qui sont normalement produits pendant la germination.

18. Procédé selon l'une quelconque des revendications 1 à 17, **caractérisé en ce que** la germination est facultativement arrêtée par au moins un moyen comprenant chauffage, séchage, broyage, séparation, extraction, compression et filtration et en récupérant subséquemment avec ou sans méthodes de traitement en aval au moins l'une des protéines hétérologues.

19. Procédé selon l'une quelconque des revendications 1 à 18 pour la production d'une composition dérivée de graines, **caractérisé en ce que** la composition est une composition sensiblement sans contaminant comprenant une ou plusieurs protéines hétérologues synthétisées dans les cotylédons d'une graine transgénique ou un jeune plant.

20. Procédé selon la revendication 19, **caractérisé en ce que** la composition comprend de plus un milieu environnant.

21. Procédé selon la revendication 19 ou 20, **caractérisé en ce que** la composition est fournie sous une forme séchée ou traitée en aval.

22. Procédé selon l'une quelconque des revendications 19 à 21, **caractérisé en ce que** la composition comprend de plus un ou plusieurs ingrédients applicables de formulation.


### SEQUENCE LISTING

<110> UniCrop Ltd
<120> A Process for converting Storage Reserves of Dicot Seeds into compositions comprising One or More Gene Products
<130> 9H08PC-EPA
<150> FI 19992659
   <151> 1999-12-10
<150> PCT/FI00/01081
   <151> 2000-12-08
<150> EP00987501.4
   <151> 2000-12-08
<160> 30
<170> PatentIn version 3.3
<210> 1
   <211> 13
   <212> DNA
   <213> Artificial
<220>
   <223> 3' primer
<400> 1
   tttttttttt tca 13
<210> 2
   <211> 13
   <212> DNA
   <213> Artificial
<220>
   <223> 3' primer
<400> 2
   tttttttttt tct 13
<210> 3
   <211> 13
   <212> DNA
   <213> Artificial
<220>
   <223> 3' primer
<400> 3
   tttttttttt tcc 13
<210> 4
   <211> 13
   <212> DNA
   <213> Artificial
<220>
   <223> 3' primer
<400> 4
   tttttttttt tcg 13
<210> 5
   <211> 13
   <212> DNA
   <213> Artificial
<220>
   <223> 3' primer
<400> 5
   tttttttttt tga 13
<210> 6
   <211> 13
   <212> DNA
   <213> Artificial
<220>
   <223> 3' primer
<400> 6
   tttttttttt tgt 13
<210> 7
   <211> 13
   <212> DNA
   <213> Artificial
<220>
   <223> 3' primer
<400> 7
   tttttttttt tgc 13
<210> 8
   <211> 13
   <212> DNA
   <213> Artificial
<220>
   <223> 3' primer
<400> 8
   tttttttttt tgg 13
<210> 9
   <211> 13
   <212> DNA
   <213> Artificial
<220>
   <223> 3' primer
<400> 9
   tttttttttt taa 13
<210> 10
   <211> 13
   <212> DNA
   <213> Artificial
<220>
   <223> 3' primer
<400> 10
   tttttttttt tat 13
<210> 11
   <211> 13
   <212> DNA
   <213> Artificial
<220>
   <223> 3' primer
<400> 11
   tttttttttt tac 13
<210> 12
   <211> 13
   <212> DNA
   <213> Artificial
<220>
   <223> 3' primer
<400> 12
   tttttttttt tag 13
<210> 13
   <211> 49
   <212> DNA
   <213> Artificial
<220>
   <223> 5' primer
<400> 13
   cgcggatccc acgggtttgt ttaccgtgag cacggaagca cccccggat 49
<210> 14
   <211> 49
   <212> DNA
   <213> Artificial
<220>
   <223> 3' primer for cloning of the rbc CDNA-clone
<400> 14
   aaggaaaaaa gcggccgcaa tttttttttt tttttttttt tttttttgc 49
<210> 15
   <211> 150
   <212> DNA
   <213> Brassica campestris
<400> 15
   ttcgcgttgt aagacatttc ataaataata tctacctcat ttcatttcca tttgtctgtt 60
   ttctttggct ttttgtttct gaggcatgtt atatcggatt gtcaagtgtc tgatttatga 120
   acaacatgta atctctatat gcatatttct 150
<210> 16
   <211> 139
   <212> DNA
   <213> Brassica campestris
<400> 16
   ttcgctttca tataataata tcttcctcat ttcatttcca ataagtctgt ttcttttttc 60
   tctttggatt tctgttacga gactttctat atcggattgt aaaatgtctg attttatgaa 120
   catgtaattt cggcaaata 139
<210> 17
   <211> 22
   <212> DNA
   <213> Artificial
<220>
   <223> bnrb1
<400> 17
   gaattctaac gacccttttc cg 22
<210> 18
   <211> 34
   <212> DNA
   <213> Artificial
<220>
   <223> UTR2
<400> 18
   ggccacactt gacaatccga tataacatgc ctca 34
<210> 19
   <211> 38
   <212> DNA
   <213> Artificial
<220>
   <223> bnrb3
<400> 19
   aaaaagcttc tagacccttt tccgtcataa gttttata 38
<210> 20
   <211> 36
   <212> DNA
   <213> Artificial
<220>
   <223> RbSiB
<400> 20
   caggtctccc atgcagctaa ctcttcctcc gttgct 36
<210> 21
   <211> 22
   <212> DNA
   <213> Artificial
<220>
   <223> RBNCO
<400> 21
   gaggaagcca tggctacttc tt 22
<210> 22
   <211> 43
   <212> DNA
   <213> Artificial
<220>
   <223> Oligo T primer
<220>
   <221> misc_feature
   <222> (42)..(42)
   <223> v is a, c, or g
<220>
   <221> mist_feature
   <222> (43)..(43)
   <223> n is a, c, g, or t
<400> 22
   cacacacagc ggccgctttt tttttttttt tttttttttt tvn 43
<210> 23
   <211> 30
   <212> DNA
   <213> Artificial
<220>
   <223> The end of the last exon of rubisco small subunit
<400> 23
   cacacacatc gacgatcatc ggattcgaca 30
<210> 24
   <211> 180
   <212> DNA
   <213> Brassica campestris
<400> 24
   ttaattcgcg ttgtaagaca tttcataaat aatatctact catttcattt ccatttgtct 60
   gttttctttg gctttttgtt tctgaggcat gttatatcgg attgtcaagt gtctgattta 120
   tgaacatgta atctctatat tgcatatttt cttcttggaa aaaaaaaaaa aaaaaaaaaa 180
<210> 25
   <211> 180
   <212> DNA
   <213> Brassica campestris
<400> 25
   ttaatttgct atgacattca cataataatc tctgctcatt tcatttccaa ttgtctgttt 60
   cttttccctt tggttttctg tttctcagac attctatatc ggattgtcaa atgtgtgatt 120
   gtgaacatgt aatctctata ttgcttcttc gtcttggtaa aaaaaaaaaa aaaaaaaaaa 180
<210> 26
   <211> 176
   <212> DNA
   <213> Brassica campestris
<400> 26
   ttaattcgct ttcatataat aatatcttct catttcattt ccaataagtc tgtttctttt 60
   tttctctttg gatttctgtt acgagacttt ctatatcgga ttgtaaaatg tctgatttta 120
   tgaacatgta atttctaaaa aaaaaaaaaa aaaaaaaaaa aaaaaaaaaa aaaaaa 176
<210> 27
   <211> 38
   <212> DNA
   <213> Artificial
<220>
   <223> bnrb4
<400> 27
   ggccatgaat tctaacgacc cttttccgtc ataaaagt 38
<210> 28
   <211> 42
   <212> DNA
   <213> Artificial
<220>
   <223> 56r6
<400> 28
   cgcgatatag aaattacatg ttcataaaat cagacatttt ac 42
<210> 29
   <211> 48
   <212> DNA
   <213> Artificial
<220>
   <223> bnrb5
<400> 29
   gaggtacccg cggccgcgaa ttctaacgac ccttttccgt cataaaag 48
<210> 30
   <211> 46
   <212> DNA
   <213> Artificial
<220>
   <223> 56r7
<400> 30
   gagaattcgg cgcgccatag aaattacatg ttcataaaat cagaca 46